# FASCICULE DE BREVET EUROPEEN

(11) **EP 1 350 464 B1**
(45) Date de publication et mention de la délivrance du brevet: **29.10.2008**
(21) Numéro de dépôt: 03290828.7
(22) Date de dépôt: 02.04.2003
(51) Int. Cl.: A61B 5/103

(54) **Procédé d'evaluation de la sensibilité la peau**
Verfahren zur Beurteilung der Empfindlichkeit der Haut
Method for evaluating the skin sensitivity

(30) Priorité: 03.04.2002 FR 0204142
(43) Date de publication de la demande: 08.10.2003
(73) Titulaire: L'ORÉAL, 75008 Paris (FR)
(72) Inventeur: Querleux, Bernard, 94170 le Perreux (FR); Burnod, Yves, 94240 l'Hay-les-Roses (FR)
(74) Mandataire: Tanty, François

(56) Documents cités:
- EP-A- 0 906 752
- WO-A-01/74240
- FR-A- 2 759 588

## Description

La présente invention concerne, entre autres, le traitement des peaux sensibles et l'évaluation de la sensibilité de la peau.

WO 01/74240 décrit un procédé permettant de tester des produits analgésiques ayant des effets sur le cerveau. Ce procédé utilise une méthode d'imagerie cérébrale.

Plus de la majorité des femmes considère avoir la peau sensible. Toutefois, pour certains dermatologues, cette appréciation est parfois davantage liée à des considérations d'ordre psychologique qu'à une réalité physiologique.

Cela rend difficile l'évaluation de l'efficacité des produits et traitements destinés aux peaux sensibles. Cela démontre également l'existence d'un besoin pour bénéficier d'un procédé d'évaluation de la sensibilité de la peau permettant de valider d'une manière objective l'appréciation émise par un individu.

L'invention est telle que définie dans les revendications ci-jointes.

Il est aussi décrit un procédé pour permettre de vérifier l'efficacité d'un produit cosmétique censé avoir une action sur la sensibilité de la peau, comportant les étapes suivantes :
- effectuer un stimulus prédéterminé apte à provoquer une réaction cérébrale caractéristique d'une peau sensible,
- recueillir par une méthode d'imagerie cérébrale une première information relative à la réaction d'au moins une région prédéterminée du cerveau en réponse à ce stimulus,
- recueillir par une méthode d'imagerie cérébrale une deuxième information relative au comportement de cette région prédéterminée du cerveau, après application ou administration du produit,
- délivrer, au moins en fonction des première et deuxième informations une troisième information relative à l'action du produit sur la sensibilité de la peau.

Avant de recueillir la deuxième information, un nouveau stimulus de la peau peut être effectué. Cela peut permettre par exemple de mettre en évidence des comportements différents du cerveau en réponse à un stimulus donné, en présence ou non d'une action du produit. Cette dernière peut être considérée comme étant démontrée par exemple lorsque l'activation cérébrale en réponse à un stimulus générateur d'inconfort est moindre après application ou administration du produit qu'auparavant, le produit ayant par exemple un effet sur la transmission du stimulus au cerveau en réduisant la sensibilité des terminaisons nerveuses de la peau. La troisième information précitée peut être délivrée par exemple sur la base d'une comparaison des première et deuxième informations.

Le produit peut être appliqué ou administré après avoir recueilli la première information. Il peut aussi, si son action est lente à s'exercer, être appliqué ou administré juste avant que la première information soit recueillie, c'est-à-dire avant que le produit ait eu le temps de produire ses effets.

Toujours selon un exemple de mise en oeuvre du procédé, le stimulus peut comporter l'application sur la peau d'une substance irritante, contenant par exemple de l'acide lactique ou de la capsaïcine.

La région prédéterminée du cerveau peut comprendre le cortex contralatéral sensoriel primaire, le cortex préfrontal supérieur et/ou la région cingulaire.

La méthode d'imagerie utilisée peut être une méthode d'imagerie par résonance magnétique fonctionnelle, encore appelée IRMf. Des méthodes d'imagerie cérébrale sont déjà utilisées pour mettre en évidence une réaction cérébrale provoquée par une application de capsaïcine. On pourra se reporter par exemple aux articles BRAIN PROCESSING OF CAPSAICINE-INDUCED SECONDARY HYPERALGESIA: A FUNCTIONAL MRI STUDY, publié dans NEUROLOGY du 11 août 1999 ; 53(3):548-57 et à l'article FUNCTIONAL REORGANIZATION OF THE HUMAN PRIMARY SOMATOSENSORY CORTEX AFTER ACUTE PAIN DEMONSTRATED BY MAGNETOENCEPHALOGRAPHY, publié dans NEUROSCI LETT du 9 février 2001 ; 298(3):195-8. D'autres méthodes d'imagerie peuvent encore être utilisées, notamment les méthodes d'imagerie par caméra à émission de positons, les méthodes d'imagerie EEG, MEG ou optique.

Le produit dont on cherche à vérifier l'efficacité peut être un produit destiné à être appliqué sur la peau, par exemple une crème, une lotion, une pommade, une huile, une poudre, cette liste n'étant pas limitative.

Le produit peut également être contenu dans un support à appliquer sur la peau, par exemple un patch, un pansement, un bandage ou un masque.

Le produit est un produit cosmétique. Au sens de la présente invention, un produit cosmétique est un produit tel que défini par la directive 93/35/CEE du Conseil du 14 juin 1993, modifiant, pour la sixième fois, la directive 76/768/CEE, et un traitement cosmétique effectué au moyen d'un produit cosmétique tel que défini plus haut.

A titre d'exemple de produit ayant une action sur la sensibilité de la peau, on peut citer par exemple, les antagonistes de substance p et notamment les extraits d'au moins un végétal de la famille des rosacées tel que décrit dans la demande de brevet européen EP 0 906 752.

Le produit peut ne pas être destiné uniquement à diminuer la sensibilité de la peau. Le produit peut ainsi être destiné, par exemple, principalement au maquillage, à l'hydratation, à la protection de la peau, notamment solaire, à la dépigmentation ou à la réparation de la peau, et avoir par ailleurs des effets sur la sensibilité de la peau. Le produit peut ainsi contenir différents composés, notamment des actifs autres que des actifs destinés à diminuer la sensibilité de la peau.

Parmi les actifs pouvant être utilisés pour diminuer la sensibilité de la peau, ceux ayant une action sur les terminaisons nerveuses peuvent être utilisés. Des actifs produisant une sensation de bien-être, adoucissants ou apaisants peuvent aussi être utilisés.

L'information relative au comportement du cerveau peut être recueillie de diverses manières. Il peut s'agir par exemple de données brutes ou ayant subi un traitement préalable afin par exemple de quantifier l'intensité et/ou l'étendue et/ou la cinétique de l'activation d'une ou plusieurs aires cérébrales.

Il est aussi décrit un procédé de commercialisation d'un produit cosmétique, comportant les étapes suivantes :
- vérifier par la mise en oeuvre du procédé défini ci-dessus l'action du produit sur la sensibilité de la peau,
- faire état, lors de la commercialisation du produit, par exemple dans la publicité ou sur un emballage, du fait que l'efficacité du produit a été vérifiée par une méthode d'imagerie cérébrale.

Il est aussi décrit une méthode pour promouvoir la vente d'un produit, notamment cosmétique, comportant l'étape consistant à faire état du fait que l'efficacité du produit a été vérifiée par une méthode d'imagerie cérébrale.

Une telle promotion du produit pourra se faire par n'importe quel canal de communication.

Elle pourra être faite notamment par un vendeur directement sur le point de vente, par la radio, la télévision ou le téléphone, notamment dans le cadre de spots publicitaires ou de messages courts. Elle pourra être faite également par le canal de la presse écrite ou par le biais de tout autre document, en particulier à des fins publicitaires. Elle pourra se faire également par Internet, par tout autre réseau informatique adéquat ou par un réseau de téléphonie mobile. Elle pourra être faite également directement sur le produit, notamment sur son packaging ou sur toute notice explicative qui lui est associée.

L'invention, a pour objet un procédé d'évaluation de la sensibilité de la peau, comportant les étapes suivantes :
- recueillir une première information relative à la sensibilité de la peau d'au moins un individu, cette première information étant recueillie par une méthode autre qu'une méthode d'imagerie cérébrale,
- effectuer au moins un stimulus prédéterminé de la peau de l'individu et recueillir, par une méthode d'imagerie cérébrale, une deuxième information relative à la réaction d'au moins une région prédéterminée du cerveau en réponse à ce stimulus,
- générer une troisième information en fonction du degré de corrélation entre les première et deuxième informations. Cette troisième information peut comporter la validation ou non de la première information, par exemple.

Le stimulus précité peut comporter l'application sur la peau d'une substance irritante, contenant par exemple de l'acide lactique ou de la capsaïcine.

La région prédéterminée du cerveau peut comprendre le cortex contralatéral sensoriel primaire, le cortex préfrontal supérieur et/ou la région cingulaire.

Dans ce procédé d'évaluation, la première information peut être obtenue par exemple grâce à un questionnaire.

L'invention a encore pour objet un procédé de sélection d'individus en vue de leur faire subir des tests, notamment en vue de l'élaboration ou de l'évaluation de produits cosmétiques, comportant l'étape consistant à soumettre ces individus au procédé d'évaluation tel que défini plus haut.

Il est aussi décrit un procédé de commercialisation d'un produit, notamment cosmétique, comportant les étapes suivantes :
- effectuer un test en appliquant un produit sur la peau d'au moins un individu qui a été reconnue comme étant sensible par la mise en oeuvre du procédé d'évaluation précité,
- faire état, lors de la commercialisation du produit cosmétique, du fait que le test a été effectué sur un individu dont on a vérifié qu'il avait une peau sensible grâce au procédé d'évaluation précité.

Il est aussi décrit une méthode pour promouvoir la vente d'un produit, notamment cosmétique, comportant l'étape consistant à faire état du fait que le test précité a été effectué sur un individu dont on a vérifié qu'il avait une peau sensible grâce au procédé d'évaluation précité.

Il est aussi décrit un procédé d'aide à la formulation ou de sélection d'un produit cosmétique, comportant les étapes suivantes :
- effectuer en relation avec un produit cosmétique un stimulus sur un individu,
- recueillir par une méthode d'imagerie cérébrale au moins une information représentative de la réaction d'au moins une région prédéterminée du cerveau, en réponse à ce stimulus,
- déterminer des modifications éventuelles à apporter à la formulation du produit en fonction de l'information précédemment recueillie ou effectuer en fonction de cette information une sélection parmi plusieurs produits.

Il est ainsi par exemple possible de formuler ou de sélectionner le produit cosmétique de façon à obtenir, lors de son application, une réaction cérébrale correspondant au mieux au but poursuivi, par exemple une sensation de bien-être ou d'apaisement.

Dans un exemple de mise en oeuvre de l'invention, un test est réalisé en soumettant un groupe d'individus à un questionnaire comportant des questions permettant d'évaluer la sensibilité de la peau des personnes du groupe.

Les questions peuvent être diverses et notamment faire référence à des facteurs climatiques ou topiques générateurs d'inconfort, par exemple la sensibilité au froid, aux brusques variations de température et aux eaux calcaires. Le questionnaire peut éventuellement comporter également des questions relatives à l'inconfort provoqué au contact de certains matériaux ou lors de l'application de certains produits.

Dans l'exemple considéré, le traitement des réponses au questionnaire permet de sélectionner au moins deux sous-groupes de personnes, un premier sous-groupe étant constitué de personnes que l'on peut considérer comme ayant la peau sensible et un deuxième sous-groupe de personnes que l'on peut considérer comme n'ayant pas la peau sensible. Chaque sous-groupe comporte par exemple neuf personnes.

Dans une première étape, on peut procéder à l'acquisition d'images anatomiques du cerveau de toutes les personnes.

Dans une deuxième étape, un produit irritant, contenant une substance génératrice d'inconfort telle que par exemple de l'acide lactique ou de la capsaïcine mélangée à un véhicule, par exemple une solution saline, peut être appliqué sur un ensemble de personnes issues de chaque sous-groupe et la réaction cérébrale de ces personnes peut être observée par une méthode d'imagerie cérébrale, par exemple l'imagerie fonctionnelle par résonance magnétique nucléaire IRMf. Des tests témoins peuvent également être effectués avec application d'une substance inerte, qui peut être le véhicule seul. Le produit irritant peut être appliqué par exemple sur une moitié du visage, et plus particulièrement on peut appliquer sur l'aile droite du nez le produit irritant et sur l'aile gauche du nez le véhicule seul.

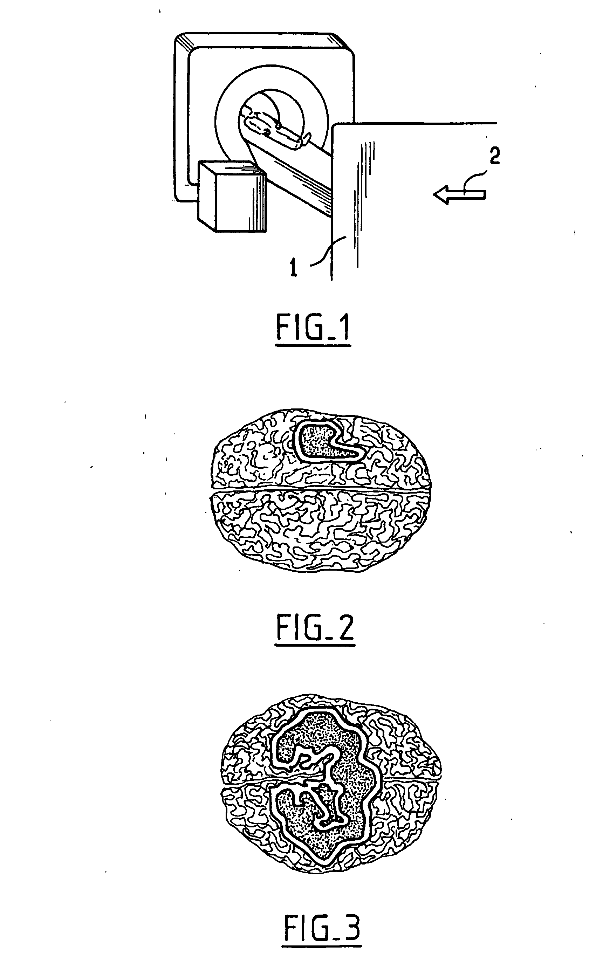

Lors de l'acquisition des images cérébrales par IRMF, chaque personne est, comme représenté schématiquement à la figure 1, allongée dans l'appareil avec un miroir lui permettant d'observer un écran 1 sur lequel est projeté une flèche 2 qui peut pointer soit vers la gauche soit vers la droite.

Chaque personne dispose également d'un clavier qui lui permet de signaler si elle ressent une sensation d'inconfort sur l'aile gauche ou l'aile droite du nez, selon la direction dans laquelle pointe la flèche. On acquiert pendant dix minutes des images de l'activité cérébrale du cerveau toutes les 3 secondes et toutes les 16 secondes on change de manière aléatoire le sens de la flèche 2 affichée.

Chaque personne peut rentrer au clavier une réponse comprise entre 0 et 3 selon le niveau d'inconfort ressenti.

La figure 2 est un exemple d'image cérébrale pour une personne considérée comme n'ayant pas la peau sensible et la figure 3 un exemple d'image cérébrale pour une personne considérée comme ayant la peau sensible.

Une activation cérébrale peut être observée au niveau du cortex contralatéral sensoriel primaire en réponse à l'application de la substance irritante parmi les personnes du premier sous-groupe tandis qu'aucune activation similaire n'est observée lors des tests témoins. Une activation cérébrale étendue, localisée notamment dans le cortex supérieur préfrontal et dans la région cingulaire, peut être observée parmi les personnes du premier sous-groupe, alors que l'activation est moindre parmi les personnes du deuxième sous-groupe, mettant ainsi en évidence la corrélation existant entre les réponses au questionnaire et la réalité physiologique.

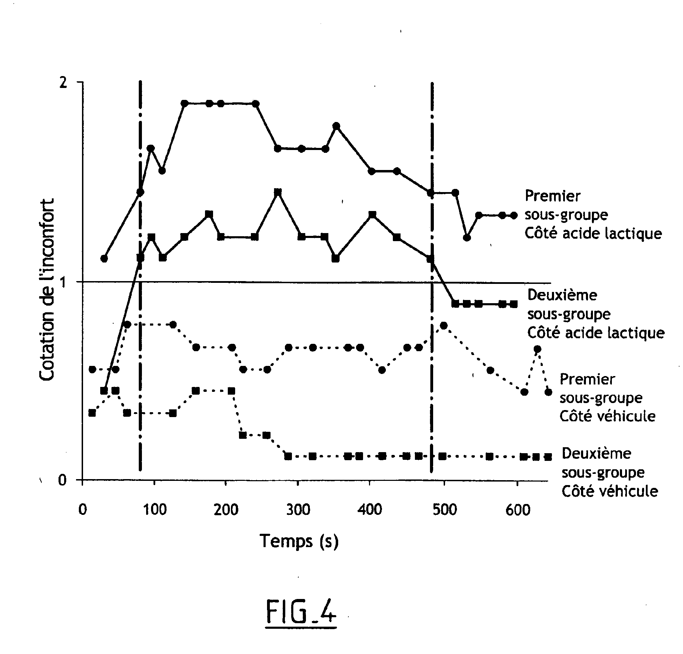

On a reporté sur la figure 4 les valeurs d'inconfort en fonction du temps, recueillies pour les personnes des premier et deuxième sous-groupes, selon que la flèche pointe du côté où a été appliqué l'acide lactique ou du côté où a été appliqué la véhicule seul.

On peut constater que l'inconfort ressenti est plus important, entre les phases de début et de fin de l'expérience, délimitées par des lignes verticales discontinues, pour les personnes ayant la peau sensible.

Dans toute la description, y compris les revendications, l'expression « comportant un » doit être comprise comme étant synonyme de « comportant au moins un».

## Revendications

1. Procédé d'évaluation de la sensibilité de la peau, comportant les étapes suivantes :
- recueillir une première information relative à la sensibilité de la peau d'au moins un individu, cette première information étant recueillie par une méthode autre qu'une méthode d'imagerie cérébrale,
- effectuer au moins un stimulus prédéterminé de la peau de l'individu et recueillir, par une méthode d'imagerie cérébrale, une deuxième information relative à la réaction d'au moins une région prédéterminée du cerveau en réponse à ce stimulus,
- générer une troisième information en fonction du degré de corrélation entre les première et deuxième informations.

2. Procédé selon la revendication 1, **caractérisé par le fait que** la troisième information comporte la validation ou non de la première information.

3. Procédé selon la revendication 1 ou 2, **caractérisé par le fait que** le stimulus comporte l'application sur la peau d'une substance irritante.

4. Procédé selon la revendication précédente, **caractérisé par le fait que** la substance irritante contient un composé choisi parmi les suivants : acide lactique, capsaïcine.

5. Procédé selon l'une quelconque des revendications précédentes, **caractérisé par le fait que** la région prédéterminée comprend le cortex contralatéral sensoriel primaire, le cortex préfrontal supérieur et/ou la région cingulaire.

6. Procédé selon l'une quelconque des revendications précédentes, **caractérisé par le fait que** la deuxième information est recueillie par une méthode d'imagerie par résonance magnétique fonctionnelle.

7. Procédé selon l'une quelconque des revendications précédentes **caractérisé par le fait que** la deuxième information est recueillie par une méthode d'imagerie par caméra à émission de positons, par une méthode d'imagerie EEG, une méthode d'imagerie MEG ou une méthode d'imagerie optique.

8. Procédé selon l'une quelconque des revendications précédentes, **caractérisé par le fait que** la première information est obtenue grâce à un questionnaire.

## Claims

1. A method of evaluating the sensitivity of the skin, comprising:
- gathering a first piece of information relating to the sensitivity of the skin of at least one individual, said first piece of information being gathered by a technique other than a brain imaging technique;
- applying at least one predetermined stimulus to the skin of the individual and using a brain imaging technique to gather a second piece of information relating to the reaction of at least one predetermined region of the brain in response to said stimulus;
- generating a third piece of information as a function of the degree of correlation between the first and second pieces of information.

2. A method according to claim 1, **characterized by** the fact that the third piece of information comprises confirming or not confirming the first piece of information.

3. A method according to claim 1 or claim 2, **characterized by** the fact that the stimulus comprises applying an irritating substance to the skin.

4. A method according to the preceding claim, **characterized by** the fact that the irritating substance contains a compound selected from the following compounds: lactic acid, capsaicin.

5. A method according to any preceding claim, **characterized by** the fact that the predetermined region comprises the primary sensory contralateral cortex, the superior prefrontal cortex, and/or the cingulate region.

6. A method according to any preceding claim, **characterized by** the fact that the second piece of information is collected by a functional magnetic resonance imaging technique.

7. A method according to any preceding claim, **characterized by** the fact that the second piece of information is gathered by an imaging technique by means of a positron-emission camera, an EEG imaging technique, an MEG imaging technique, or an optical imaging technique.

8. A method according to any preceding claim, **characterized by** the fact that the first piece of information is obtained by means of a questionnaire.

## Patentansprüche

1. Verfahren zur Beurteilung der Empfindlichkeit der Haut, umfassend die folgenden Schritte:
- eine erste Information über die Empfindlichkeit der Haut mindestens einer Person gewonnen, wobei diese erste Information durch eine andere Methode als eine Hirnbildgebungsmethode gewonnen wird,
- mindestens einen vorbestimmten Stimulus der Haut der Person ausüben und durch eine Hirnbildgebungsmethode eine zweite Information über die Reaktion mindestens einer vorbestimmten Gehirnregion als Antwort auf den Stimulus gewinnen,
- eine dritte Information in Abhängigkeit von dem Grad der Korrelation zwischen der ersten und der zweiten Information erzeugen.

2. Verfahren nach Anspruch 1, **dadurch gekennzeichnet, dass** die dritte Information die Validierung der ersten Information umfasst oder nicht umfasst.

3. Verfahren nach Anspruch 1 oder 2, **dadurch gekennzeichnet, dass** der Stimulus den Auftrag einer reizenden Substanz auf die Haut umfasst.

4. Verfahren nach dem vorhergehenden Anspruch, **dadurch gekennzeichnet, dass** die reizende Substanz eine Verbindung enthält, die aus den folgenden ausgewählt ist: Milchsäure, Capsaicin.

5. Verfahren nach einem der vorhergehenden Ansprüche, **dadurch gekenntzeichnet, dass** die vorbestimmte Region den primären sensoriellen kontralateralen Kortex, den oberen präfrontalen Kortex und/oder die zinguläre Region umfasst.

6. Verfahren nach einem der vorhergehenden Ansprüche, **dadurch gekennzeichnet, dass** die zweite Information durch eine Methode der funktionellen Magnetresonanzbildgebung gewonnen wird.

7. Verfahren nach einem der vorhergehenden Ansprüche, **dadurch gekennzeichnet, dass** die zweite Information durch eine Methode der Bildgebung mit Hilfe der Positronenemissionskamera, durch eine EEG-Bildgebungsmethode, eine MEG-Bildgebungsmethode oder eine Methode der optischen Bildgebung gewonnen wird.

8. Verfahren nach einem der vorhergehenden Ansprüche, **dadurch gekennzeichnet, dass** die erste Information mit Hilfe eines Fragebogens erhalten wird.
